(19) Europäisches Patentamt
European Patent Office
Office européen des brevets

(11) **EP 4 393 453 A1**

(12) **EUROPEAN PATENT APPLICATION**
published in accordance with Art. 153(4) EPC

(43) Date of publication:
**03.07.2024 Bulletin 2024/27**

(21) Application number: **22875587.2**

(22) Date of filing: **01.08.2022**

(51) International Patent Classification (IPC):
**A61F 2/28** (2006.01)    **A61K 9/19** (2006.01)
**A61K 38/39** (2006.01)    **A61L 27/00** (2006.01)
**A61L 27/22** (2006.01)    **A61P 19/00** (2006.01)
**B65D 81/32** (2006.01)    **C07K 14/78** (2006.01)

(52) Cooperative Patent Classification (CPC):
**A61F 2/28; A61K 9/19; A61K 38/39; A61L 27/00;
A61L 27/22; A61P 19/00; B65D 81/32; C07K 14/78**

(86) International application number:
**PCT/JP2022/029561**

(87) International publication number:
**WO 2023/053710 (06.04.2023 Gazette 2023/14)**

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**
Designated Extension States:
**BA ME**
Designated Validation States:
**KH MA MD TN**

(30) Priority: **30.09.2021  JP 2021162131**

(71) Applicant: **FUJIFILM Corporation
Tokyo 106-8620 (JP)**

(72) Inventors:
• **FUKUNAGA, Kazuto**
**Ashigarakami-gun, Kanagawa 258-8577 (JP)**
• **KATO, Nobuhiko**
**Ashigarakami-gun, Kanagawa 258-8577 (JP)**

(74) Representative: **Hoffmann Eitle
Patent- und Rechtsanwälte PartmbB
Arabellastraße 30
81925 München (DE)**

Remarks:
The complete document including Reference
Table(s) and the Sequence Listing(s) can be
downloaded from the EPO website

(54) **TISSUE RESTORATIVE MATERIAL KIT AND TISSUE RESTORATION METHOD**

(57)    The application provides a tissue repair material kit and a tissue repair method, where the tissue repair material kit includes a cylindrical container that has an opening portion having an opening diameter smaller than an inner diameter of the container and a tissue repair material, in which in a case where the tissue repair material is supplied, a storage elastic modulus of the tissue repair material at 25°C is 0.1 kPa to 100 kPa.

FIG. 1

EP 4 393 453 A1

**Description**

**BACKGROUND OF THE INVENTION**

1. Field of the Invention

The present disclosure relates to a tissue repair material kit and a tissue repair method.

2. Description of the Related Art

A tissue repair material is used to form tissue (for example, a bone) in a tissue-deficient part (for example, a bone-deficient part) caused by, for example, injuries, surgery, or the like. A bone tissue repair material, which is one of such tissue repair materials, is disclosed in, for example, JP2018-094401A.

**SUMMARY OF THE INVENTION**

Techniques related to tissue repair, including the technique of JP2018-094401A, have been studied in the related art. However, at present, a technique for supplying a tissue repair material to a narrow tissue-deficient part is not sufficient.

The present disclosure has been made in consideration of such circumstances, and an object to be achieved by an embodiment of the present disclosure is to provide a tissue repair material kit that makes it possible to easily supply a tissue repair material to a narrow tissue-deficient part.

An object to be achieved by another embodiment of the present disclosure is to provide a tissue repair method using the tissue repair material kit.

The present disclosure includes the following aspects.

<1> A tissue repair material kit comprising:

a cylindrical container that has an opening portion having an opening diameter smaller than an inner diameter of the container; and
a tissue repair material,
in which in a case where the tissue repair material is supplied, a storage elastic modulus of the tissue repair material at 25°C is 0.1 kPa to 100 kPa.

<2> The tissue repair material kit according to <1>, further comprising a discharge part that has an inner diameter smaller than the inner diameter of the cylindrical container and is provided at the opening portion.
<3> The tissue repair material kit according to <1> or <2>, in which the opening diameter of the opening portion or an inner diameter of an discharge part is 10% to 70% of the inner diameter of the cylindrical container.
<4> The tissue repair material kit according to any one of <1> to <3>, in which the opening diameter of the opening portion or an inner diameter of an discharge part is 5 mm or less.
<5> The tissue repair material kit according to any one of <1> to <4>, in which the tissue repair material has a particulate shape in a dry state, and the opening diameter of the opening portion or an inner diameter of an discharge part is 40% to 300% of a maximum particle diameter of the tissue repair material in the dry state.
<6> The tissue repair material kit according to any one of <1> to <5>, further comprising:

a pressurization unit,
wherein the opening portion is provided at one end of the cylindrical container, and
the pressurization is provided at the other end of the cylindrical container.

<7> The tissue repair material kit according to <6>, in which the pressurization unit is a plunger.
<8> The tissue repair material kit according to any one of <1> to <7>, in which the tissue repair material is accommodated in the cylindrical container.
<9> The tissue repair material kit according to any one of <1> to <8>, in which a water absorption rate of the tissue repair material is 300% to 2,000%.
<10> The tissue repair material kit according to any one of <1> to <9>, in which in a case where the tissue repair material contains a water-containing liquid, the storage elastic modulus of the tissue repair material at 25°C is 0.1 kPa to 100 kPa in a case where a value obtained by multiplying a mass fraction of a content of the water-containing liquid with respect to a dry mass of the tissue repair material by a tap density of the tissue repair material in a dry state is 0.40 g/mL to 0.70 g/mL.

<11> The tissue repair material kit according to any one of <1> to <10>, further comprising a water-containing liquid for mixing with the tissue repair material.

<12> The tissue repair material kit according to <11>, in which a content of the water-containing liquid is 50% by mass to 2,000% by mass with respect to a dry mass of the tissue repair material.

<13> The tissue repair material kit according to any one of <1> to <12>, in which the tissue repair material contains a biocompatible polymer.

<14> The tissue repair material kit according to <13>, in which the biocompatible polymer is a recombinant peptide.

<15> The tissue repair material kit according to <14>, in which the recombinant peptide includes the following peptide of (A), (B), or (C),

(A) a peptide consisting of an amino acid sequence set forth in SEQ ID NO: 1

(B) a peptide consisting of an amino acid sequence, in which one or several amino acid residues in the amino acid sequence set forth in SEQ ID NO: 1 are modified, and having biocompatibility

(C) a peptide consisting of an amino acid sequence which has a partial sequence having 80% or more sequence identity with a partial amino acid sequence consisting of 4th to 192nd amino acid residues in the amino acid sequence set forth in SEQ ID NO: 1, and having biocompatibility,

SEQ ID NO: 1:

GAP(GAPGLQGAPGLQGMPGERGAAGLPGPKGERGDAGPKGADGAPGAPGL
QGMPGERGAAGLPGPKGERGDAGPKGADGAPGKDGVRGLAGPIGPPGERGAAGLPG
PKGERGDAGPKGADGAPGKDGVRGLAGPIGPPGPAGAPGAPGLQGMPGERGAAGLP
GPKGERGDAGPKGADGAPGKDGVRGLAGPP)3G.

<16> The tissue repair material kit according to any one of <1> to <15>, in which the tissue repair material is a bone tissue repair material.

<17> A tissue repair method comprising:

a step of supplying the tissue repair material to a tissue-deficient part by using the tissue repair material kit according to any one of <1> to <16>.

According to the embodiment of the present disclosure, there is provided a tissue repair material kit capable of easily supplying a tissue repair material to a narrow tissue-deficient part.

According to another embodiment of the present disclosure, a tissue repair method using the tissue repair material kit is provided.

**BRIEF DESCRIPTION OF THE DRAWINGS**

Fig. 1 is a schematic view showing an example of a tissue repair material kit.

Fig. 2 is a schematic view showing a syringe assembled by using the tissue repair material kit of Fig. 1.

Fig. 3 is a schematic view showing an example of the tissue repair material kit.

Fig. 4 is a schematic view showing an example of the tissue repair material kit.

**DESCRIPTION OF THE PREFERRED EMBODIMENTS**

In the present disclosure, a numerical range described using "to" includes numerical values before and after "to" as a minimum value and a maximum value, respectively.

In the numerical ranges described stepwise in the present disclosure, the upper limit value or the lower limit value described in one numerical range may be replaced with the upper limit value or the lower limit value of the numerical range described stepwise in other stages. Further, in a numerical range described in the present disclosure, the upper limit value or the lower limit value of the numerical range may be replaced with the value shown in Examples.

In the present disclosure, in a case where a plurality of substances corresponding to each component in a material is present, the amount of each component in the material means the total amount of the plurality of substances present in the material, unless otherwise specified.

In the present disclosure, a combination of two or more preferred aspects is a more preferred aspect.

In the present disclosure, the term "step" includes not only an independent step but also a step that cannot be clearly distinguished from other steps, as long as the intended purpose of the step is achieved.

<Tissue repair material kit>

A tissue repair material kit according to the present disclosure includes a cylindrical container that has an opening portion having an opening diameter smaller than an inner diameter of the container and a tissue repair material, in which in a case where the tissue repair material is supplied, a storage elastic modulus of the tissue repair material at 25°C is 0.1 kPa to 100 kPa.

A tissue repair material may be used for repairing a tissue-deficient part. However, a tissue-deficient part where tissue repair is required may be narrow, and there is a case where it is difficult to supply a tissue repair material to the tissue-deficient part. As described above, for example, although Patent Document 1 discloses a bone tissue repair material as a tissue repair material, it has not considered a method of supplying the tissue repair material to a narrow tissue-deficient part.

On the other hand, the tissue repair material kit according to the present disclosure includes a cylindrical container that has an opening portion having an opening diameter smaller than an inner diameter of the container and a tissue repair material. In addition, the storage elastic modulus of the tissue repair material at 25°C is 0.1 kPa to 100 kPa in a case of being supplied, that is, the tissue repair material is easily deformed, and thus the tissue repair material accommodated in the cylindrical container can be easily supplied to a narrow tissue-deficient part through the opening portion.

The phrase "in a case where a tissue repair material is supplied" means a case where the tissue repair material in the cylindrical container is supplied to the tissue-deficient part through the opening portion or a discharge part (described later).

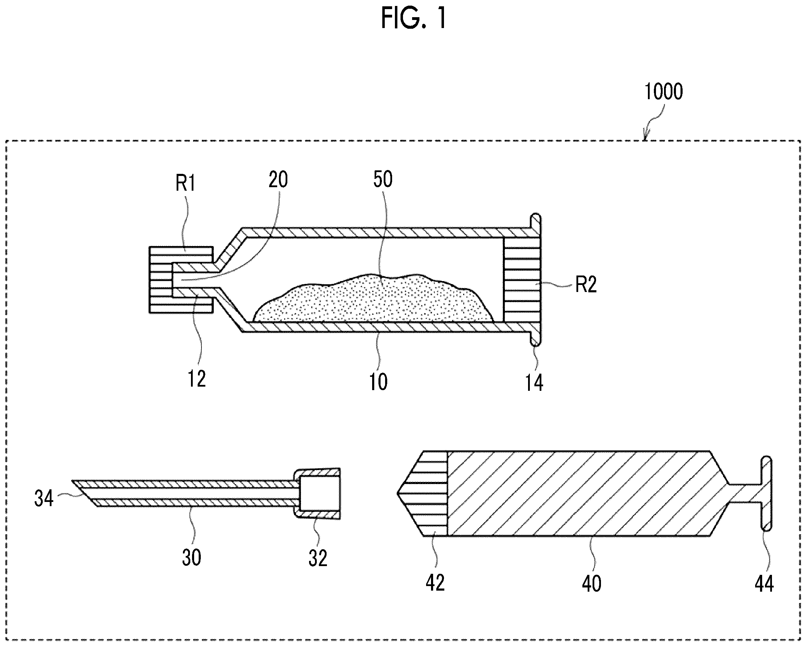

A tissue repair material kit 1000 shown in Fig. 1 as an example includes a cylindrical container 10 having an opening portion 20 having an opening diameter smaller than an inner diameter of the container, a tissue repair material 50, a discharge part 30, and a plunger 40. The opening portion 20 having one end of the cylindrical container 10 and the other end (the end part opposite to the opening portion 20) of the cylindrical container 10 are respectively sealed with rubber stoppers R1 and R2.

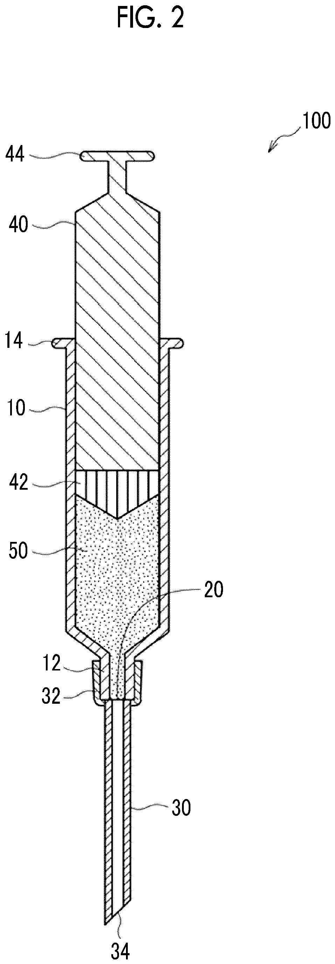

In a case of assembling the tissue repair material kit 1000, it is possible to obtain a syringe 100 shown in Fig. 2 as an example. In another aspect, the tissue repair material kit 1000 may be the syringe 100 itself assembled as described above.

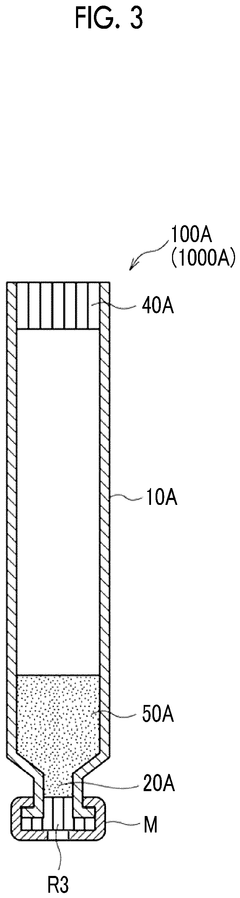

A tissue repair material kit 1000A shown in Fig. 3 as another example is a cartridge 100A that includes a cylindrical container 10A having an opening portion 20A having an opening diameter smaller than an inner diameter of the container, a tissue repair material 50A, and a plunger 40A. The opening portion 20A provided at one end (an end part opposite to the plunger 40) of the cylindrical container 10A is sealed with a rubber stopper R3. The cylindrical container 10A and the rubber stopper R3 are fixed by an aluminum member M.

The cartridge as shown in Fig. 3 can be used, for example, as a cartridge of a known syringe, similarly to a cartridge of an anesthetic which is used in a syringe for dental anesthesia.

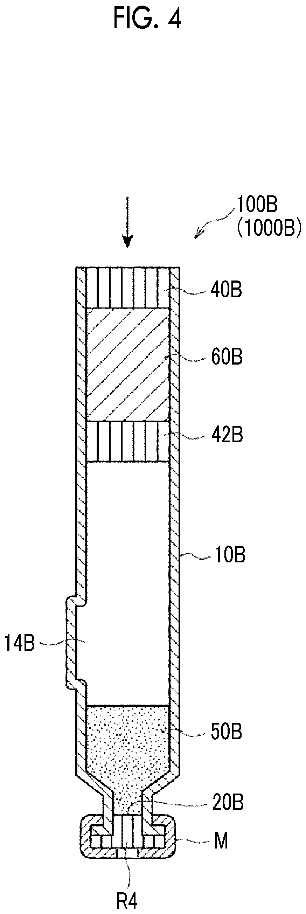

A tissue repair material kit 1000B shown in Fig. 4 as another example of the cartridge is a cartridge 100B that includes a cylindrical container 10B having an opening portion 20B having an opening diameter smaller than an inner diameter of the container, a tissue repair material 50B, plungers 40B and 42B, and a water-containing liquid 60B held between the plunger 40B and the plunger 42B. The opening portion 20B provided at one end (an end part opposite to the plunger 40B) of the cylindrical container 10B is sealed with a rubber stopper R4, and The cylindrical container 10B and the rubber stopper RB are fixed by the aluminum member M.

A bypass 14B is provided in the cylindrical container 10B, and in a case where the plungers 40B and 42B are pressed toward the direction of the arrow and the water-containing liquid 60B reaches the bypass 14B, the water-containing liquid 60B passes through the bypass 14B and flows to the tissue repair material 50B side. This makes it possible to mix the tissue repair material 50B and the water-containing liquid 60B.

In another aspect, a filter that allows the water-containing liquid 60B to communicate with the tissue repair material 50B side may be provided in the plunger 42B, whereby the water-containing liquid 60B can be allowed to flow out to the tissue repair material 50B side through the filter of the plunger 42B without passing through the bypass 14B.

Hereinafter, each configuration will be described in more detail.

[Cylindrical container]

The cylindrical container has a cylindrical container having an opening portion having an opening diameter smaller than an inner diameter of the container.

The cylindrical container is a container having a cavity therein. The shape of the cylindrical container is not particularly limited. For example, it may be an elongated rod shape, and it is preferably a cylinder or a square cylinder and particularly preferably a cylinder. In addition, for example, a protruding portion including an opening portion may be integrally provided in the cylindrical container.

The cylindrical container may have a member for facilitating operation. For example, the cylindrical container has a syringe shape, and it may have, for example, a finger flange 14 as shown in Fig. 1 in order to facilitate the operation.

The material of the cylindrical container is not particularly limited, and it may be, for example, a resin, a metal, or glass. From the viewpoint of visibility, the material of the cylindrical container preferably includes a transparent resin, glass, or the like.

The inner diameter of the cylindrical container means a diameter of a circle having the same area as the area of the portion of the cavity part in the cross section perpendicular to the longitudinal direction of the cylindrical container. In a case where the inner diameter of the cylindrical container is not uniform in the longitudinal direction, the maximum value thereof is defined as the inner diameter of the cylindrical container.

The inner diameter of the cylindrical container is, for example, a value that is measured by a projection image measuring instrument, a laser displacement meter, or a caliper.

The inner diameter of the cylindrical container is, for example, preferably 5 mm to 50 mm, and it is more preferably 5 mm to 30 mm from the viewpoint of handleability (ease of holding and the like).

The length of the cylindrical container (not including the opening portion) in the longitudinal direction is not particularly limited. However, it is, for example, preferably 70 mm to 200 mm, and it is more preferably 70 mm to 120 mm from the viewpoint of handleability.

The opening portion is a portion that allows the tissue repair material accommodated in the cylindrical container to flow out of the cylindrical container. The opening diameter of the opening portion means a diameter of a circle having the same area as the area of the opening portion in a case of being viewed in a plan view from the outside of the cylindrical container. In a certain aspect, the opening portion may function as a portion that supplies a tissue repair material to a tissue-deficient part.

The opening diameter of the opening portion is, for example, a value that is measured by a projection image measuring instrument, a laser displacement meter, or a caliper.

From the viewpoint of reducing the force required to allow the tissue repair material to flow out of the cylindrical container, the opening diameter of the opening portion is preferably 10% to 70% and more preferably 20% to 70% or less of the inner diameter of the cylindrical container.

The opening diameter of the opening portion may be, for example, 15 mm or less. As described above, the opening portion can function as a portion that supplies a tissue repair material to a tissue-deficient part. However, in this aspect, from the viewpoint of more easily supplying the tissue repair material to a narrow tissue-deficient part, the opening diameter of the opening portion is preferably 0.8 mm to 10 mm and more preferably 0.8 mm to 6.6 mm. The upper limit of the opening diameter of the opening portion is still more preferably 5 mm or less. The opening diameter of the opening portion is even still more preferably 0.8 mm to 2.5 mm.

As will be described later, the tissue repair material may have a particulate shape in a dry state. In this aspect, From the viewpoint of reducing the force required to allow the tissue repair material to flow out of the cylindrical container, the opening diameter of the opening portion is preferably 40% to 300% of the maximum particle diameter of the tissue repair material in a dry state. As described above, the opening portion can function as a portion that supplies a tissue repair material to a tissue-deficient part. However, in this aspect, from the viewpoint of more easily supplying the tissue repair material to a narrow tissue-deficient part, the opening diameter of the opening portion is more preferably 60% to 200%. A measuring method for the maximum particle diameter will be described later.

The description that the tissue repair material is in a "dry state" is used to mean a state where the tissue repair material does not contain a liquid (for example, a water-containing liquid described below); however, moisture due to humidity in the air may be contained.

The position of the opening portion is not particularly limited and may be appropriately determined in consideration of the use application of the tissue repair material kit and the like. For example, the opening portion may be provided at one end of the cylindrical container. In this aspect, for example, a protruding portion having an inner diameter smaller than the inner diameter of the container may be integrally provided at one end of the cylindrical container, and an end part of the protruding portion may be the opening portion.

The cylindrical container may have a connecting portion (for example, a connecting portion 12 as shown in Fig. 1) for connection to a discharge part described later. In this aspect, the opening portion may be provided at an end part of the connecting portion.

The connecting portion may be integrated with the cylindrical container or may be a member that is separate from the cylindrical container.

The material of the connecting portion container is not particularly limited, and it may be, for example, a resin, a metal, or glass. From the viewpoint of visibility, the material of the cylindrical container preferably includes a transparent resin, glass, or the like.

[Discharge part]

The tissue repair material kit may include a discharge part that has an inner diameter smaller than the inner diameter of the cylindrical container and is provided at the opening portion. The discharge part guides the tissue repair material that has flowed out through the opening portion of the cylindrical container and allows the tissue repair material to flow out from the hole portion (for example, a hole portion 34 in Fig. 1) provided in the discharge part.

The discharge part is a member having a cavity therein and is subjected to fluid communication with the opening portion of the cylindrical container. The shape of the discharge part is not particularly limited. For example, it may be an elongated rod shape, and it is preferably a cylinder or a square cylinder and particularly preferably a cylinder. In addition, for example, the discharge part may be bent. In addition, the discharge part may have a rod shape that tapers from one end toward the other end in the longitudinal direction.

The inner diameter of the discharge part means a diameter of a circle having the same area as the area of the portion of the cavity part in the cross section perpendicular to the longitudinal direction of the discharge part. It means a diameter of a circle having the same area as the area of the hole portion through which the tissue repair material flows out from the outside of the discharge part in a case where the hole portion is viewed in a plan view, in a case where the inner diameter of the discharge part is not uniform in the longitudinal direction. For example, in a case where the discharge part (and the cavity part) has a rod shape that tapers from one end toward the other end (the hole portion from which the tissue repair material flows out) in the longitudinal direction, the inner diameter of the discharge part means the diameter of the hole portion of the tapered tip part, where the inner diameter has the minimum value.

The opening diameter of the discharge part is, for example, a value that is measured by a projection image measuring instrument, a laser displacement meter, or a caliper.

From the viewpoint of reducing the force required to allow the tissue repair material to flow out of the cylindrical container, the inner diameter of the discharge part is preferably 10% to 70% and more preferably 20% to 70% or less of the inner diameter of the cylindrical container.

In addition, since a short container which is not bulky can be obtained by making the cylindrical container thicker than the discharge part, the inner diameter of the discharge part is still more preferably 20% to 60% of the inner diameter of the cylindrical container.

The inner diameter of the discharge part may be, for example, 0.5 mm to 15 mm. From the viewpoint of more easily supplying the tissue repair material to a narrow tissue-deficient part, the inner diameter of the discharge part is preferably 0.8 mm to 10 mm and more preferably 0.8 mm to 6.6 mm. The upper limit of the inner diameter of the discharge part is still more preferably 5 mm or less. The inner diameter of the discharge part is even still more preferably 0.8 mm to 2.5 mm.

The inner diameter of the discharge part is, for example, a value that is measured by a projection image measuring instrument, a laser displacement meter, or a caliper.

As will be described later, the tissue repair material may have a particulate shape in a dry state. In this aspect, from the viewpoint of reducing the force required to allow the tissue repair material to flow out of the cylindrical container, the inner diameter of the discharge part is preferably 40% to 300% of the maximum particle diameter of the tissue repair material in a dry state. From the viewpoint of more easily supplying the tissue repair material to a narrow tissue-deficient part, the inner diameter of the discharge part is more preferably 60% to 200%. A measuring method for the maximum particle diameter will be described later.

The aspect in which the tissue repair material kit includes the discharge part is not particularly limited. The discharge part may be separated from the cylindrical container or may be connected to the cylindrical container through a connecting portion provided in the cylindrical container. For example, in the example shown in Fig. 1, the connecting portion 12 may be inserted into and connected to an insertion part 32 of the discharge part 30.

The material of the discharge part is not particularly limited; however, it may be, for example, a resin, a metal, or glass. From the viewpoint of visibility, the material of the cylindrical container preferably includes a transparent resin, glass, or the like.

[Pressurization unit]

The tissue repair material kit may include a pressurization unit for pushing out the tissue repair material in the cylindrical container from the opening portion or the discharge part.

For example, in an aspect in which the cylindrical container has an opening portion at one end, the tissue repair material kit may include a pressurization unit that is provided at the other end of the cylindrical container.

The pressurization unit is not particularly limited, and examples thereof include a plunger, a piston, and compressed air. Among these, from the viewpoint of facilitation, the pressurization unit is preferably a plunger.

The plunger is a member that slides on an inner wall of the cylindrical container to push out the tissue repair material accommodated in the cylindrical container to the outside of the cylindrical container. The plunger may include a gasket (for example, a gasket 42 shown in Fig. 1) for improving airtightness with respect to the inner wall of the cylindrical container and may include a pusher for entering the inside of the cylindrical container or exiting therefrom (for example, a pusher 44 shown in Fig. 1). The plunger may be a plunger that is driven by the pressure of the medium or the like. The material of the gasket is not particularly limited. For example, synthetic rubber can be used, where a lubricant may be provided. The material of the pusher is not particularly limited, and it may be, for example, a resin, a metal, or glass. In addition, the plunger may be a plunger that also serves as a gasket (for example, the plunger 40A of Fig. 3 or the plungers 40B and 42B of Fig. 4), and in this case, it is preferable to be made of rubber.

The aspect in which the tissue repair material kit includes the pressurization unit is not particularly limited. For example, the pressurization unit may be in a state of being separated from the cylindrical container or may be in a state of being provided in advance in the cylindrical container.

[Tissue repair material]

The tissue repair material is a material that contributes to the formation of tissue at the implantation site by being implanted in a living body, and it may include or may not include cells. In addition, the tissue repair material may contain or may not contain a component which promotes a reaction in a living body, such as a growth factor, a drug, or the like. In addition, a tissue repair material, which has been mixed or subjected to form a composite with an inorganic material such as hydroxyapatite, may be applied. Further, the tissue repair material is not necessarily a tissue repair material that contributes to the formation of the normal tissue which is normally present in a tissue-deficient part (for example, an implantation site) and a tissue repair material that also contains a material which promotes the formation of the abnormal tissue including the scar tissue and the like.

The tissue repair material may be, for example, a bone tissue repair material. The bone tissue repair material is a material that is supplied to a bone-deficient part of a living body or a site in which bone morphogenesis is desired, and due to having a certain biocompatibility and biodegradability, the strength thereof is ensured for a desired period of time. It contributes to the formation of the bone tissue while maintaining the volume of the supply site, thereby being capable of being a place of replacement with the formed bone tissue. As a result, it is presumed that the bone tissue repair material is arranged, whereby the formation of the tissue such as the bone proceeds favorably. However, the present disclosure is not restricted by the theory of this presumption. It is preferable that the bone tissue repair material has sufficient biodegradability and bone replaceability to replace the bone tissue.

The shape of the tissue repair material is not particularly limited, and it may be, for example, a particulate shape or a block shape. The particulate shape means a shape of a granule, a powder, a slurry, or the like.

The shape of the granule is not particularly limited, and it may be, for example, an irregular shape, a spherical shape, a powdery shape, a porous shape, a fibrous shape, a spindle shape, a flat shape, a sheet shape, or the like. The shape of the granule is preferably an irregular shape, a spherical shape, a powdery shape, or a porous shape. The "irregular shape" means a shape having a non-uniform surface, and examples thereof include a shape having unevenness, such as a shape of rock.

In a case where the tissue repair material has a particulate shape, the particle diameter of the tissue repair material means the size of each particle. The maximum particle diameter is a maximum value of the sizes of a plurality of particles. The size of a grain is defined as a square root of a projected area, that is, a length of one side of a square having the same area as the projected area of the particle. The projected area is calculated as an area of a region corresponding to the tissue repair material by carrying out image processing to binarize an image captured by placing a grain on a background of a brightness different from that of the particle. Examples of the analysis apparatus that can be used for imaging and image processing include "Morphologi 3G" (Spectris plc).

The aspect in which the tissue repair material kit includes the tissue repair material is not particularly limited. For example, the tissue repair material may be provided in the tissue repair material kit in a state of being accommodated in a packaging material or the like, and the tissue repair material may be accommodated in the cylindrical container in a case of using the tissue repair material kit.

In another aspect, the tissue repair material may be accommodated in the cylindrical container in advance, which is preferable from the viewpoint of workability.

From the viewpoint of improving the fluidity of the tissue repair material, the water absorption rate of the tissue repair material is preferably 300% to 2,000%, more preferably 400% to 2,000%, and particularly, still more preferably 500% to 2,000%.

It is preferable that the tissue repair material has a water absorption rate equal to or higher than a certain level. For example, in a case of a tissue repair material containing gelatin granules (for example, a bone tissue repair material), it is preferable to exhibit a water absorption rate of 300% or more. In a case where the water absorption rate is set to 300% or more, it is easy to obtain favorable tissue repair ability. From the viewpoint of holding blood clotting during tissue repair, the water absorption rate of the tissue repair material is preferably 400% or more and more preferably 500% or more. The upper limit of the water absorption rate of the tissue repair material is not particularly limited; however, it is preferably 2,000% or less. In a case where the tissue repair material contains only gelatin granules, the water absorption rate of the tissue repair material is defined as the water absorption rate of the gelatin granules.

The water absorption rate of the tissue repair material means physical properties that are measured as follows. In each of three filter cups of which the tare mass has been measured in advance (each having a volume of 500 $\mu$L, which includes a filter having a pore diameter of 0.22 $\mu$m on the bottom surface; hereinafter, referred to as a container), 10.0 mg of each test substance (tissue repair material) is collected (n = 3). A sufficient amount of water is added thereto, followed by mixing until the water absorption by the test substance is saturated (rotation: 2 hours, at an environmental temperature). Next, centrifugation (6,000 $\times$ g, 1 minute, 25°C) is carried out to remove excess water, and the mass of the container containing the test substance after water absorption (the total mass after water absorption) is measured. Separately, a blank test is carried out three times in the same manner as described above except that the test substance is not used, and a value obtained by subtracting the tare mass of the container from the mass of the container, to which water has adhered, is defined as the residual water amount. The average value from the three times of measurements of the residual water amount is defined as the residual water amount of the blank test, and a value obtained by subtracting the residual water amount and the tare mass of the container from the total mass after the water absorption is defined as the mass of the test substance after water absorption. The mass of the test substance after water absorption is divided by the mass of the test substance before water absorption to define the water absorption rate (%).

The tissue repair material kit may include a water-containing liquid that is to be mixed with the tissue repair material. The water-containing liquid may be, for example, a solution or a dispersion liquid, which uses water as a solvent.

The water-containing liquid is not particularly limited, and it may be, for example, water for injection, saline, a hyaluronic acid aqueous solution, a carboxymethyl cellulose aqueous solution, a polyethylene glycol aqueous solution, a growth factor aqueous solution, an antibiotic aqueous solution, blood, a bone marrow fluid, a platelet-rich blood plasma, or a mixture thereof.

The aspect in which the tissue repair material kit includes the water-containing liquid is not particularly limited. For example, the water-containing liquid may be provided in the tissue repair material kit in a state of being accommodated in a packaging material or the like, and the water-containing liquid may be mixed with the tissue repair material in a case of using the tissue repair material kit.

In another aspect, the water-containing liquid may be mixed with the tissue repair material in advance.

The content of the water-containing liquid is preferably 50% by mass to 2,000% by mass and more preferably 100% by mass to 2,000% by mass with respect to the dry mass of the tissue repair material. A state where the water-containing liquid is mixed with the tissue repair material and thus the mass of the tissue repair material is increased may be referred to as a hydrous state.

In a case where a tissue repair material is supplied, (that is, a case where the tissue repair material in the cylindrical container is supplied to the tissue-deficient part through the opening portion or a discharge part), the storage elastic modulus of the tissue repair material at 25°C is 100 kPa or less, and it is more preferably 50 kPa or less. This makes the tissue repair material easily deformed in a case where the tissue repair material is supplied, which prevents the tissue repair material from being bridged and clogged at the opening portion or an upper part of the discharge part or prevents the tissue repair materials from being pushed with each other to be crushed and destroyed.

The lower limit of the storage elastic modulus of the tissue repair material at 25°C is not particularly limited from the viewpoint of the above-described effect; however, it is set to 0.1 kPa or more from the viewpoint of handleability.

In addition, from the viewpoint of easily maintaining the shape of the tissue repair material supplied to the tissue-deficient part, the storage elastic modulus is preferably 0.1 kPa to 50 kPa.

The storage elastic modulus is measured as follows. A test substance is placed in a SUS ring having an inner diameter of 15 mm and a height of 7.5 mm, a vibratory flow is applied thereto in a shear direction while carrying out pressing with a constant load of 1 N by using a disposable parallel plate ($\Phi$12 mm) of a rheometer (Anton Paar, MCR302), and a linear viscoelasticity measurement is carried out. The amount of the test substance is set such that the height thereof in a case of being pressed is 3.5 mm or more and 4.5 mm or less. The angular frequency is swept from 1 rad/sec to 50 rad/sec and then swept from 50 rad/sec to 1 rad/sec, and a storage elastic modulus at 25°C at 1 rad/sec in the latter sweep is measured (n = 2 to 3).

Under the following conditions, the storage elastic modulus of the tissue repair material at 25°C is preferably 100 kPa or less and more preferably 50 kPa or less. This makes it easy to reduce the force required to allow the tissue repair material to flow out of the cylindrical container. Under the following conditions, the lower limit of the storage elastic modulus of the tissue repair material at 25°C is not particularly limited from the viewpoint of the above-described effect; however, it is preferably set to 0.1 kPa or more from the viewpoint of handleability.

[Conditions]

In a case where the tissue repair material contains the water-containing liquid, a value obtained by multiplying a mass fraction of a content of the water-containing liquid with respect to a dry mass of the tissue repair material by a tap density of the tissue repair material in a dry state is 0.40 g/mL to 0.70 g/mL.

The tap density is measured as follows. A test substance is placed up to 5 mL in a 10 mL graduated cylinder of which the tare mass has been measured in advance, the mass of the graduated cylinder containing the test substance is weighed, and a value obtained by subtracting the tare mass is defined as the mass of the test substance. This graduated cylinder is tapped 50 times or more until the volume of the test substance becomes constant, and the scale of the graduated cylinder is read to measure the volume after the tapping. The mass of the test substance is divided by the volume after tapping to obtain the tap density (g/mL).

The tissue repair material may contain a biocompatible polymer.

The "polymer" is a molecule having a large molecular weight and refers to a molecule having a structure including a large number of repetitions of a unit that is obtained substantially or conceptually from a molecule having a small molecular weight. The "polymer" is preferably a compound having a weight-average molecular weight of 10,000 or more. Examples of the polymer include a polyamine, a polysaccharide, a polypeptide, a protein, a polyamide, a polyester, a polyolefin, a polyether, and a polynucleotide.

The "biocompatibility" means a property that does not cause a significantly harmful response such as a long-term and chronic inflammatory reaction, during contact with a living body. Examples of the substance having biocompatibility include a protein and polysaccharides.

From the viewpoint of being used as a tissue repair material, the tissue repair material preferably contains a biodegradable polymer. Examples of the biodegradable polymer include polypeptides such as a naturally derived peptide, a recombinant peptide, and a chemically synthesized peptide (for example, gelatin described later). In addition, examples thereof include polylactic acid, polyglycolic acid, a lactic acid/glycolic acid copolymer (PLGA), hyaluronic acid, glycosaminoglycan, proteoglycan, chondroitin, cellulose, agarose, carboxymethylcellulose, chitin, and chitosan. Among the above, It is particularly preferable that the tissue repair material in the present embodiment contains a recombinant peptide. Examples of the non-biodegradable polymer include polytetrafluoroethylene (PTFE), polyurethane, polypropylene, polyester, vinyl chloride, polycarbonate, acryl, silicone, and 2-methacryloyloxyethyl phosphoryl choline (MPC).

The tissue repair material is preferably manufactured from a biocompatible polymer, more preferably manufactured from recombinant gelatin, and particularly preferably manufactured from CBE3 (set forth in SEQ ID NO: 1).

The kind of the polypeptide such as a recombinant peptide or a chemically synthesized peptide is not particularly limited as long as the polypeptide has biocompatibility and biodegradability. Examples of such a polypeptide include gelatin, collagen, elastin, fibronectin, ProNectin, laminin, tenascin, fibrin, fibroin, entactin, thrombospondin, and Retro-Nectin. Among these, the tissue repair material in the present embodiment preferably contains gelatin, collagen, or atelocollagen, more preferably contains natural gelatin, recombinant gelatin, or chemically synthesized gelatin, and still more preferably contains recombinant gelatin. The natural gelatin referred to herein means gelatin produced from naturally derived collagen.

Examples of the natural gelatin and the recombinant gelatin thereof include gelatins derived from animals such as a fish and a mammal, where gelatin derived from a mammalian animal is preferable. Examples of the mammalian animal include a human, a horse, a pig, a mouse, and a rat. In a case where the tissue repair material according to the present embodiment contains recombinant gelatin, it is more preferable that the recombinant gelatin is derived from a human.

Hereinafter, an amino acid sequence constituting a polypeptide is expressed by using one-letter notation (for example, "G" in a case of a glycine residue) or three-letter notation (for example, "Gly" in a case of a glycine residue), which is well known in the art. In addition, unless otherwise specified, "%" regarding an amino acid sequence of a polypeptide is based on the number of amino acid (or imino acid) residues.

The recombinant gelatin means a polypeptide or a protein-like substance which has an amino acid sequence similar to that of gelatin produced according to a gene recombination technique. The following recombinant gelatin is implanted in a living body, whereby it can be used as a tissue repair material which contributes to the formation of tissue at the implanted site. The "tissue" that can be repaired by the tissue repair material may be hard tissue such as teeth and bone, and the following recombinant gelatin is particularly suitable as a bone tissue repair material.

The recombinant gelatin preferably has a repetition of a sequence represented by Gly-X-Y which is characteristic of collagen. Here, a plurality of pieces of Gly-X-Y may be the same or different from each other. In Gly-X-Y, Gly represents a glycine residue, and X and Y represent any amino acid residue other than the glycine residue. It is preferable that X and Y include a large amount of imino acid residues, that is, proline residues or oxyproline residues. The content of such imino acid residues preferably occupies 10% to 45% of the entire gelatin. The content of Gly-X-Y in the gelatin is preferably 80% or more, more preferably 95% or more, and still more preferably 99% or more with respect to the entire gelatin.

For example, recombinant gelatin disclosed in EP1014176A, US6992172B, WO2004/85473A, WO2008/103041A, JP2010-519293A, JP2010-519252A, JP2010-518833A, JP2010-519251A, WO2010/128672A, WO2010/147109A, and the like can be used as the recombinant gelatin, the recombinant gelatin is not limited thereto.

The molecular weight of the recombinant gelatin is preferably 2 kDa to 100 kDa, more preferably 5 kDa to 90 kDa or less, and still more preferably has a molecular weight of 10 kDa to 90 kDa.

In addition, from the viewpoint of biocompatibility, the recombinant gelatin is preferably recombinant gelatin that further contains a cell adhesion signal and the recombinant gelatin is more preferably recombinant gelatin that has, in one molecule, two or more cell adhesion signals which are present in the recombinant gelatin. Examples of such a cell adhesion signal include sequences such as an RGD sequence, a YIGSR sequence, a PDSGR sequence, an LGTIPG sequence, an IKVAV sequence, and an HAV sequence. Among these, an RGD sequence is preferable, and an ERGD sequence among the RGD sequences is more preferable.

However, it is preferable that the sequence of the recombinant gelatin satisfies at least one of the following aspects (1-1) to (1-3). It is noted that the recombinant gelatin may singly have the following aspects (1-1) to (1-3) or may have a combination of two or more of the aspects.

(1-1) It does not include a serine residue and a threonine residue.
(1-2) It does not include a serine residue, a threonine residue, an asparagine residue, a tyrosine residue, and a cysteine residue.
(1-3) It does not include an amino acid sequence represented by Asp-Arg-Gly-Asp.

It is preferable that the recombinant gelatin has a repeating structure of A-[(Gly-X-Y)$_n$]$_m$-B. m represents an integer of 2 to 10, and it is preferably 3 to 5. A and B represent any amino acid or amino acid sequence. n represents an integer of 3 to 100, and it is preferably 15 to 70 and more preferably 50 to 60.

It is more preferable that the recombinant gelatin is represented by a formula: Gly-Ala-Pro-[(Gly-X-Y)$_{63}$]$_3$-Gly. In the formula, 63 pieces of X's each independently represent any amino acid residue, and 63 pieces of Y's each independently represent any amino acid residue. It is noted that 3 pieces of [(Gly-X-Y)$_{63}$] may be the same or different from each other.

It is preferable that the recombinant gelatin satisfies at least one of the following aspects (2-1) to (2-4). It is noted that the recombinant gelatin may singly have the following aspects (2-1) to (2-4) or may have a combination of two or more of the aspects.

(2-1) It is such that the carbamoyl group has not been hydrolyzed.
(2-2) It does not include procollagen.
(2-3) It does not include telopeptide.
(2-4) It is a substantially pure collagen-like material prepared from a nucleic acid encoding natural collagen.

From the viewpoint of high tissue repair ability, the recombinant peptide preferably includes the following peptide (A), (B), or (C).

(A) A peptide consisting of an amino acid sequence set forth in SEQ ID NO: 1.
(B) A peptide consisting of an amino acid sequence in which one or several amino acid residues in the amino acid sequence set forth in SEQ ID NO: 1 are modified (for example, deleted, substituted, or added), and having biocompatibility.
(C) A peptide consisting of an amino acid sequence which has a partial sequence having 80% or more sequence identity with a partial amino acid sequence consisting of 4th to 192nd amino acid residues in the amino acid sequence set forth in SEQ ID NO: 1, and having biocompatibility.

SEQ ID NO: 1:

GAP(GAPGLQGAPGLQGMPGERGAAGLPGPKGERGDAGPKGADGAPGAPGL
QGMPGERGAAGLPGPKGERGDAGPKGADGAPGKDGVRGLAGPIGPPGERGAAGLPG
PKGERGDAGPKGADGAPGKDGVRGLAGPIGPPGPAGAPGAPGLQGMPGERGAAGLP
GPKGERGDAGPKGADGAPGKDGVRGLAGPP)3G

In the peptide defined in (B) described above, the number of amino acid residues to be modified (for example, deleted, substituted, or added) varies depending on the total number of amino acid residues in the recombinant gelatin;

however, it is preferably 2 to 15 and more preferably 2 to 5.

In (C) described above, the "sequence identity" regarding the amino acid sequences of the two kinds of peptides to be compared (that is, the peptide of (A) and the peptide of (C)) refers to a value calculated according to the following expression. It is noted that the comparison (alignment) of a plurality of peptides is carried out according to a conventional method so that the number of identical amino acid residues is the largest.

$$\text{Sequence identity (\%)}$$
$$= [(\text{number of identical amino acid residues})/(\text{alignment length})] \times 100$$

In (C) described above, the "partial amino acid sequence consisting of 4th to 192nd amino acid residues" corresponds to a repeating unit in the amino acid sequence set forth in SEQ ID NO: 1. The "partial sequence" corresponds to a repeating unit in the sequence of (C) described above. It is sufficient that the peptide of (C) described above includes at least one repeating unit (partial sequence) having 80% or more sequence identity with the repeating unit in the amino acid sequence set forth in SEQ ID NO: 1, and it is preferable to include two or more thereof.

It is noted that in a case where the peptide of (C) described above includes a plurality of different repeating units, some of the plurality of repeating units may be such that the sequence identity with the repeating unit in the amino acid sequence set forth in SEQ ID NO: 1 is less than 80%. However, in the peptide of (C) described above, it is preferable that the total number of amino acid residues of the repeating unit (partial sequence) having 80% or more sequence identity with the repeating unit in the amino acid sequence set forth in SEQ ID NO: 1 is 80% or more of the total number of amino acid residues.

In addition, from the viewpoint of tissue repair ability, the sequence identity of the partial sequence in the peptide of (C) with the partial amino acid sequence consisting of 4th to 192nd amino acid residues in the amino acid sequence set forth in SEQ ID NO: 1 is preferably 90% or more and more preferably 95% or more.

The length of the peptide defined in (C) described above is such that the number of amino acid residues can be set to 151 to 2,260, where it is preferably 193 or more from the viewpoint of the decomposability after crosslinking, and it is preferably 944 or less from the viewpoint of stability. In addition, the number of amino acid residues is more preferably 380 to 756.

The recombinant gelatin described above can be produced by a gene recombination technique known to those skilled in the art, and it may be produced, for example, according to the method described in EP1014176A, US6992172B, WO2004/85473A, WO2008/103041A, or the like. Specifically, a gene encoding an amino acid sequence of a predetermined recombinant gelatin is obtained, the gene is incorporated into an expression vector to prepare a recombinant expression vector, and the expression vector is introduced into a proper host to prepare a transformant. The recombinant gelatin is produced by culturing the obtained transformant in an appropriate medium. Therefore, it is possible to prepare the recombinant gelatin that is used in the present disclosure by collecting the recombinant gelatin produced from a culture product.

The tissue repair material is not limited to a tissue repair material consisting of one material such as recombinant gelatin. For example, in addition to the recombinant gelatin, components which promote reactions in a living body, such as a growth factor and a drug, and other components such as cells, which can contribute to the repair or regeneration of the tissue, may be contained. Examples of such a component include a component associated with bone regeneration or osteogenesis, such as a bone-inducing agent. Examples of the bone-inducing agent include bone morphogenic factor (bone morphogenic protein; BMP) and basic fibroblast growth factor (bFGF), which are not particularly limited. In addition, a tissue repair material, which has been mixed or subjected to form a composite with an inorganic material such as hydroxyapatite, may be applied.

A manufacturing method for the tissue repair material kit is not particularly limited. For example, the tissue repair material kit may be manufactured by preparing the cylindrical container, the tissue repair material, and the like by methods that are generally used in the present technical field, and then packaging these, for example, in one package.

<Tissue repair method>

A tissue repair method according to the present disclosure includes a step of supplying the tissue repair material to a tissue-deficient part by using the tissue repair material kit according to the present disclosure.

The step of supplying the tissue repair material may be appropriately carried out in consideration of the configuration of the tissue repair material kit and the like.

For example, the tissue repair material kit 1000 shown in Fig. 1 is used to assemble the syringe 100 shown in Fig. 4. The hole portion 34 of the discharge part 30 is inserted in the vicinity of the tissue-deficient part. Then, the plunger 40 is pressed into the cylindrical container 10 to supply the tissue repair material 50 to the tissue-deficient part through the hole portion 34.

In this way, it is possible to supply the tissue repair material to a narrow tissue-deficient part.

Examples

Hereinafter, the present disclosure will be described in more detail with reference to Examples. However, the present disclosure is not limited to these Examples.

<Example 1>

(Recombinant gelatin)

In the present Example, a recombinant peptide CBE3 was used as a biocompatible polymer contained in a tissue repair material. As the CBE3, the following one described in WO2008/103041A was used.

Molecular weight: 51.6 kD
Structure: $\mathrm{GAP[(GXY)_{63}]_3G}$
Number of amino acids: 571 amino acids
RGD sequence: 12 sequences
Imino acid content: 33%
Almost 100% of amino acids have a repeating structure of GXY
CBE3 has an ERGD sequence.

The amino acid sequence of the CBE3 does not include a serine residue, a threonine residue, an asparagine residue, a tyrosine residue, and a cysteine residue.

Isoelectric point: 9.34
Hydrophilic repeating unit ratio in polymer: 26.1%
Amino acid sequence (SEQ ID NO: 1)

GAP(GAPGLQGAPGLQGMPGERGAAGLPGPKGERGDAGPKGADGAPGAPGL

QGMPGERGAAGLPGPKGERGDAGPKGADGAPGKDGVRGLAGPIGPPGERGAAGLPG

PKGERGDAGPKGADGAPGKDGVRGLAGPIGPPGPAGAPGAPGLQGMPGERGAAGLP

GPKGERGDAGPKGADGAPGKDGVRGLAGPP)3G

(Container used to freeze recombinant gelatin solution)

A cylindrical cup-shaped container made of an aluminum alloy (JIS A5056 alloy) was prepared. The container had a shape in which a side surface and a bottom surface were closed and a top surface was open in a case where a curved surface was regarded as the side surface. The bottom surface had a thickness of 5 mm, and an inner circumference of the bottom surface was chamfered with R2 mm. The insides of the side surface and the bottom surface were coated with a tetrafluoroethylene/hexafluoropropylene copolymer (FEP), and as a result, the inner diameter of the container was 104 mm. Hereinafter, this container will be referred to as a "cylindrical container".

(Preparation of tissue repair material)

A solution containing the above-described recombinant gelatin was purified and then concentrated to 4.0% by mass by ultrafiltration at 30°C. The obtained gelatin aqueous solution was lyophilized to obtain a lyophilized body 1. Water for injection was added to the lyophilized body 1, the temperature was raised to 37°C over 30 minutes, and the resultant was redissolved to obtain again a 7.5% by mass gelatin aqueous solution. Approximately 20 g of the gelatin aqueous solution was poured into cylindrical containers, and then 14 pieces of the cylindrical containers were placed, through a 1 mm-thick glass plate, on a $350 \times 634 \times 20$ mm aluminum plate precooled to approximately -35°C, covered with a lid, and allowed to stand for 1 hour to obtain a frozen body of the gelatin. The frozen body of the gelatin was lyophilized using a lyophilizer (DFR-5N-B manufactured by ULVAC, Inc.) to remove water, thereby preparing a lyophilized body 2.

The lyophilized body 2 was manually divided into a size of 20 mm square or less and pulverized using a screen mill (Comil U10, manufactured by Quadro Engineering). The pulverization was carried out twice using screens having pore diameters different from each other. In the first pulverization, a screen having a pore diameter of 0.079 inches was used as the screen, and a rotary impeller was rotated at a rotation speed of 1,100 ± 10 rpm to carry out pulverization. As the rotary impeller, a rake type (7A1611) was used. In the second pulverization, a screen having a pore diameter of 0.040 inches was used as the screen. The rotary impeller was rotated at a rotation speed of 2,200 ± 10 rpm to carry out pulverization.

After the second pulverization, granules on a sieve, which had been obtained after sieving at a sieve opening of 1.4 mm and a sieve opening of 0.3 mm, were collected, and 89.5 mg thereof was used to fill a glass vial (barrel diameter: 24.3 mm) of 10 mL was by using a filling machine (Aishin Nano Technologies Co., Ltd., TF-70AD). The same vial was installed in a clean oven (Nitto Rika Kogyo Co., Ltd., NCO-500A600L-WS) and subjected to a heating treatment in a nitrogen atmosphere at 135°C for 5 hours to obtain a specimen 1 as a granular tissue repair material. Table 1 shows the results of measuring the particle diameter, the water absorption rate, the storage elastic modulus at 25°C (hereinafter, may be simply referred to as a "storage elastic modulus"), and the tap density. For the particle diameter, 89.5 mg of the specimen 1 was subjected to measurement in a dry state using an image examination device (Morphologi G3, Spectris plc). The storage elastic modulus was measured in a state where an amount of 333% by mass of the water for injection was added to the specimen 1 and a state where an amount of 222% by mass of the water for injection was added to the specimen 1.

The storage elastic modulus was measured as follows. A test substance was placed in a SUS ring having an inner diameter of 15 mm and a height of 7.5 mm, a vibratory flow was applied thereto in a shear direction while carrying out pressing with a constant load of 1 N by using a disposable parallel plate (Φ12 mm) of a rheometer (Anton Paar, MCR302), and a linear viscoelasticity measurement was carried out. The amount of the test substance was set such that the height thereof in a case of being pressed was 3.5 mm or more and 4.5 mm or less. The angular frequency was swept from 1 rad/sec to 50 rad/sec and then swept from 50 rad/sec to 1 rad/sec, and a storage elastic modulus at 1 rad/sec in the latter sweep was measured. The above measurement was repeated three times, and the average value therefrom was defined as the storage elastic modulus of the test substance.

The tap density was measured as follows. A test substance was placed up to 5 mL in a 10 mL graduated cylinder of which the tare mass had been measured in advance, the mass of the graduated cylinder containing the test substance was weighed, and a value obtained by subtracting the tare mass was defined as the mass of the test substance. This graduated cylinder was tapped 50 times or more until the volume of the test substance became constant, and the scale of the graduated cylinder was read to measure the volume after the tapping. The mass of the test substance was divided by the volume after tapping to obtain the tap density (g/mL).

(Discharge test)

A syringe "SS-01T" (a syringe main body including a cylindrical container having an opening portion that has an opening diameter smaller than the inner diameter of the container and a plunger, inner diameter of container: 6.6 mm, opening diameter of opening portion of protruding portion: 1.2 mm, total length: 94.6 mm) manufactured by TERUMO CORPORATION was used to prepare tissue repair material kits 1 to 6.

(1) Tissue repair material kit 1

- Syringe "SS-01T" (cylindrical container, plunger)
- Specimen 1 (tissue repair material)
- 24G needle (discharge part, inner diameter of discharge part: 0.3 mm)

(2) Tissue repair material kit 2

- Syringe "SS-01T" (cylindrical container, plunger)
- Specimen 1 (tissue repair material)
- 20G needle (discharge part, inner diameter of discharge part: 0.6 mm)

(3) Tissue repair material kit 3

- Syringe "SS-01T" (cylindrical container, plunger)
- Specimen 1 (tissue repair material)
- 18G needle (discharge part, inner diameter of discharge part: 0.8 mm)

(4) Tissue repair material kit 4

- Syringe "SS-01T" (cylindrical container, plunger)
- Specimen 1 (tissue repair material)

(5) Tissue repair material kit 5

- Syringe "SS-01T" (cylindrical container, plunger) in which the opening diameter of the opening portion has been expanded to 2.5 mm with a drill.
- Specimen 1 (tissue repair material)

(6) Tissue repair material kit 6

- Syringe "SS-01T" (cylindrical container, plunger) in which the opening diameter of the opening portion has been expanded to 4.5 mm with a drill.
- Specimen 1 (tissue repair material)

In addition, the following tissue repair material kit 7 was prepared for the reference experiment.

(7) Tissue repair material kit 7 for reference experiment

- Syringe "SS-01T" (cylindrical container, plunger) in which the opening diameter of the opening portion has been expanded to 6.6 mm with a drill.
- Specimen 1 (tissue repair material)

A syringe was filled with 80.0 mg of the specimen 1, and an amount of 333% by mass of the water for injection was added to the specimen 1 to swell the specimen 1. The protruding portion is integrally connected to the cylindrical container and includes the opening portion. However, it functions as a connecting portion in a case where a needle (discharge part) is attached as in the tissue repair material kits 1 to 3. The tissue repair material kits 4 to 7 are examples in which the needle (discharge part) is not provided.
A piston of each syringe was pushed with an electric measurement stand (MX-1000N-FA-CN, manufactured by IMADA Co., Ltd.) to which a force gauge (ZP-200N, manufactured by IMADA Co., Ltd.) was attached, and the dischargeability was evaluated, and the maximum load required for discharge was measured. Table 2 shows the results of repeating the operation three times with each syringe. Those which have not been subjected to the measurement are indicated as "-".
Table 3 shows the ratio of the opening diameter of the opening portion or the inner diameter of the discharge part to the maximum particle diameter of the specimen 1 in the dry state.
In addition, Table 4 shows the ratio of the opening diameter of the opening portion or the inner diameter of the discharge part to the inner diameter of the cylindrical container.

<Comparative Example 1>

As a bone tissue repair material, β TCP granules (CURASAN, CERASORB M) were prepared. Table 1 shows the results of measuring the particle diameter, the water absorption rate, the storage elastic modulus, and the tap density in the same manner as in Example 1. The storage elastic modulus was measured in a state where an amount of 77% by mass of the water for injection was added to βTCP.

The reason why the amount of the tissue repair material and the amount of the water are different between Example 1 and Comparative Example 1 is to equalize the amount of water per unit volume of the tissue repair material in the hydrous state.

(Discharge test)

Table 2 shows the results of carrying out a discharge test in the same manner as in Example 1. In addition, Table 3 shows the ratios of the inner diameter of the discharge part and the opening diameter of the opening portion to the maximum particle diameter of the βTCP granules in the dry state.

<Comparative Example 2>

A specimen 1 was prepared as a bone tissue repair material. Table 1 shows the results of measuring the storage elastic modulus in the same manner as in Example 1 except that the water for injection was not added to the specimen 1 (the particle diameter and the water absorption rate are the same values as those in Example 1)

(Discharge test)

Table 2 shows the results of carrying out a discharge test in the same manner as in Example 1 except that the water for injection was not added to the specimen 1. It is noted that the ratios of the inner diameter of the discharge part and the opening diameter of the opening portion to the maximum particle diameter in the dry state are the same as those in Example 1, and thus the description thereof is omitted in Table 3.

[Table 1]

|  | Particle diameter in dry state ($\mu$m) | | | Storage elastic modulus (kPa) | Water absorption rate (%) | Tap density (g/mL) |
|---|---|---|---|---|---|---|
|  | Minimum value (reference) | Medium value (reference) | Maximum value |  |  |  |
| Example 1: specimen 1 (with water addition) | 13.2 | 1220 | 1900.5 | 46 (333% by mass, water added) 41 (222% by mass, water added) | 558 | 0.199 |
| Comparative Example 1: βTCP granule | 13.0 | 957.4 | 1269.5 | 1999 (77% by mass, water added) | 99 | 0.859 |
| Comparative Example 2: specimen 1 (without water addition) | 13.2 | 1220 | 1900.5 | 529 (without water addition) | 558 | 0.199 |

[Table 2]

|  | Force required for discharge (N) | | | | |
|---|---|---|---|---|---|
| Opening diameter of opening portion or inner diameter of discharge part | 0.8 mm | 1.2 mm | 2.5 mm | 4.0 mm | 6.6 mm (reference) |

(continued)

|  | Force required for discharge (N) | | | | |
|---|---|---|---|---|---|
| Example 1: specimen 1 (with water addition) | 57.9 | 12.8 | 5.4 | 4.1 | 1.8 |
| Comparative Example 1: βTCP granule | Cannot be discharged | Cannot be discharged | Cannot be discharged | Cannot be discharged | 3.0 |
| Comparative Example 2: specimen 1 (without water addition) | Cannot be discharged | Cannot be discharged | Cannot be discharged | Cannot be discharged | 3.2 |

[Table 3]

|  | Ratio of inner diameter of discharge part and opening diameter of opening portion with respect to maximum particle diameter in dry state | | | | | |
|---|---|---|---|---|---|---|
| Opening diameter of opening portion or inner diameter of discharge part | 0.3 mm | 0.6 mm | 0.8 mm | 1.2 mm | 2.5 mm | 4.0 mm |
| Example 1: specimen 1 (with water addition) | 16% | 32% | 42% | 63% | 132% | 210% |
| Comparative Example 1: βTCP granule | 24% | 47% | 63% | 95% | 197% | 315% |

[Table 4]

|  | Ratio of opening diameter of opening portion or inner diameter of discharge part with respect to inner diameter of cylindrical container | | | | | |
|---|---|---|---|---|---|---|
| Opening diameter of opening portion or inner diameter of discharge part | 0.3 mm | 0.6 mm | 0.8 mm | 1.2 mm | 2.5 mm | 4.0 mm |
| Example 1: specimen 1 (with water addition) | 5% | 9% | 12% | 18% | 38% | 61% |

In Example 1, the storage elastic modulus of the tissue repair material in a case where the tissue repair material is supplied is 41 kPa to 46 kPa, which is in the range specified by the present application, the tissue repair material is capable of being discharged at all of the opening diameters of the opening portions and the inner diameters of the discharge parts, and thus it is possible to easily supply the tissue repair material to a narrow tissue-deficient part.

In addition, as can be seen from Table 2, the pressure required for discharge is as small as 57.9 N or less in Example 1, and thus the tissue repair material can be supplied with a smaller force.

In addition, as can be seen from Table 4, in a case where the opening diameter of the opening portion and the inner diameter of the discharge part are 0.8 mm to 4.0 mm, the ratio of the diameter to the inner diameter of the cylindrical container is 12% to 61%, which is within a preferred range. From the results shown in Table 2, it can be seen that the tissue repair material can be supplied with a smaller force.

On the other hand, in Comparative Example 1 and Comparative Example 2, the storage elastic moduli of the tissue repair material in a case where the tissue repair material is supplied are 1,999 kPa and 558 kPa, respectively, the tissue repair material can not be discharged from a discharge part having an inner diameter smaller than that of the cylindrical container but can be discharged only from the opening portion having an inner diameter equal to the inner diameter of the cylindrical container (see the result of the 6.6 mm of the inner diameter of the discharge part, which is described as the reference value in Table 2).

The disclosure of JP2021-162131 filed on September 30, 2021 is incorporated in the present specification by reference in its entirety. All documents, patent applications, and technical standards described in the present specification are herein incorporated by reference to the same extent that individual documents, patent applications, and technical standards have been specifically and individually indicated to be incorporated by reference, respectively.

[Sequence list]

International application based on the International Patent Cooperation Treaty 21F01232W1JP22029561_4.xml

**Claims**

1. A tissue repair material kit comprising:

   a cylindrical container that has an opening portion having an opening diameter smaller than an inner diameter of the container; and
   a tissue repair material,
   wherein in a case where the tissue repair material is supplied, a storage elastic modulus of the tissue repair material at 25°C is 0.1 kPa to 100 kPa.

2. The tissue repair material kit according to claim 1, further comprising:
   a discharge part that has an inner diameter smaller than the inner diameter of the cylindrical container and is provided at the opening portion.

3. The tissue repair material kit according to claim 1 or 2,
   wherein the opening diameter of the opening portion or the inner diameter of the discharge part is 10% to 70% of the inner diameter of the cylindrical container.

4. The tissue repair material kit according to any one of claims 1 to 3,
   wherein the opening diameter of the opening portion or the inner diameter of the discharge part is 5 mm or less.

5. The tissue repair material kit according to any one of claims 1 to 4,

   wherein the tissue repair material has a particulate shape in a dry state, and
   the opening diameter of the opening portion or the inner diameter of the discharge part is 40% to 300% of a maximum particle diameter of the tissue repair material in the dry state.

6. The tissue repair material kit according to any one of claims 1 to 5, further comprising:

   a pressurization unit,
   wherein the opening portion is provided at one end of the cylindrical container, and
   the pressurization is provided at the other end of the cylindrical container.

7. The tissue repair material kit according to claim 6,
   wherein the pressurization unit is a plunger.

8. The tissue repair material kit according to any one of claims 1 to 7,
   wherein the tissue repair material is accommodated in the cylindrical container.

9. The tissue repair material kit according to any one of claims 1 to 8,
   wherein a water absorption rate of the tissue repair material is 300% to 2,000%.

10. The tissue repair material kit according to any one of claims 1 to 9,
    wherein in a case where the tissue repair material comprises a water-containing liquid, the storage elastic modulus of the tissue repair material at 25°C is 0.1 kPa to 100 kPa in a case where a value obtained by multiplying a mass fraction of a content of the water-containing liquid with respect to a dry mass of the tissue repair material by a tap density of the tissue repair material in a dry state is 0.40 g/mL to 0.70 g/mL.

11. The tissue repair material kit according to any one of claims 1 to 10, further comprising:
    a water-containing liquid for mixing with the tissue repair material.

12. The tissue repair material kit according to claim 11,
    wherein a content of the water-containing liquid is 50% by mass to 2,000% by mass with respect to a dry mass of the tissue repair material.

13. The tissue repair material kit according to any one of claims 1 to 12,
    wherein the tissue repair material comprises a biocompatible polymer.

14. The tissue repair material kit according to claim 13,
    wherein the biocompatible polymer is a recombinant peptide.

15. The tissue repair material kit according to claim 14,
    wherein the recombinant peptide comprises the following peptide of (A), (B), or (C):

    (A) a peptide consisting of an amino acid sequence set forth in SEQ ID NO: 1;
    (B) a peptide consisting of an amino acid sequence, in which one or several amino acid residues in the amino acid sequence set forth in SEQ ID NO: 1 are modified, and having biocompatibility; or
    (C) a peptide consisting of an amino acid sequence which has a partial sequence having 80% or more sequence identity with a partial amino acid sequence consisting of 4th to 192nd amino acid residues in the amino acid sequence set forth in SEQ ID NO: 1, and having biocompatibility:

    SEQ ID NO: 1:

    GAP(GAPGLQGAPGLQGMPGERGAAGLPGPKGERGDAGPKGADGAPGAPGL

    QGMPGERGAAGLPGPKGERGDAGPKGADGAPGKDGVRGLAGPIGPPGERGAAGLPG

    PKGERGDAGPKGADGAPGKDGVRGLAGPIGPPGPAGAPGAPGLQGMPGERGAAGLP

    GPKGERGDAGPKGADGAPGKDGVRGLAGPP)3G.

16. The tissue repair material kit according to any one of claims 1 to 15,
    wherein the tissue repair material is a bone tissue repair material.

17. A tissue repair method comprising:
    a step of supplying the tissue repair material to a tissue-deficient part by using the tissue repair material kit according to any one of claims 1 to 16.

FIG. 1

EP 4 393 453 A1

# FIG. 2

# FIG. 3

# FIG. 4

## INTERNATIONAL SEARCH REPORT

| International application No. |
|---|
| **PCT/JP2022/029561** |

**A.    CLASSIFICATION OF SUBJECT MATTER**

*A61F 2/28*(2006.01)i; *A61K 9/19*(2006.01)i; *A61K 38/39*(2006.01)i; *A61L 27/00*(2006.01)i; *A61L 27/22*(2006.01)i; *A61P 19/00*(2006.01)i; *B65D 81/32*(2006.01)i; *C07K 14/78*(2006.01)i

FI:    A61F2/28; A61L27/22; A61L27/00; A61P19/00; A61K38/39; A61K9/19; B65D81/32 Q; C07K14/78 ZNA

According to International Patent Classification (IPC) or to both national classification and IPC

**B.    FIELDS SEARCHED**

Minimum documentation searched (classification system followed by classification symbols)

A61F2/28; A61K9/19; A61K38/39; A61L27/00; A61L27/22; A61P19/00; B65D81/32; C07K14/78

Documentation searched other than minimum documentation to the extent that such documents are included in the fields searched

Published examined utility model applications of Japan 1922-1996
Published unexamined utility model applications of Japan 1971-2022
Registered utility model specifications of Japan 1996-2022
Published registered utility model applications of Japan 1994-2022

Electronic data base consulted during the international search (name of data base and, where practicable, search terms used)

**C.    DOCUMENTS CONSIDERED TO BE RELEVANT**

| Category* | Citation of document, with indication, where appropriate, of the relevant passages | Relevant to claim No. |
|---|---|---|
| X | JP 2012-130672 A (KANAGAWA ACAD. OF SCI. & TECHNOL.) 12 July 2012 (2012-07-12) paragraphs [0007], [0008], [0011], [0015], [0018], [0019] | 1-12 |
| Y |  | 13-17 |
| Y | JP 2017-42477 A (FUJIFILM CORP.) 02 March 2017 (2017-03-02) claims 5, 7, 11, paragraphs [0043], [0067] | 13-17 |

☐ Further documents are listed in the continuation of Box C.　　☑ See patent family annex.

| * | Special categories of cited documents: | "T" | later document published after the international filing date or priority date and not in conflict with the application but cited to understand the principle or theory underlying the invention |
|---|---|---|---|
| "A" | document defining the general state of the art which is not considered to be of particular relevance | | |
| "E" | earlier application or patent but published on or after the international filing date | "X" | document of particular relevance; the claimed invention cannot be considered novel or cannot be considered to involve an inventive step when the document is taken alone |
| "L" | document which may throw doubts on priority claim(s) or which is cited to establish the publication date of another citation or other special reason (as specified) | "Y" | document of particular relevance; the claimed invention cannot be considered to involve an inventive step when the document is combined with one or more other such documents, such combination being obvious to a person skilled in the art |
| "O" | document referring to an oral disclosure, use, exhibition or other means | | |
| "P" | document published prior to the international filing date but later than the priority date claimed | "&" | document member of the same patent family |

| Date of the actual completion of the international search | Date of mailing of the international search report |
|---|---|
| **30 September 2022** | **11 October 2022** |

| Name and mailing address of the ISA/JP | Authorized officer |
|---|---|
| **Japan Patent Office (ISA/JP)** **3-4-3 Kasumigaseki, Chiyoda-ku, Tokyo 100-8915 Japan** | |
| | Telephone No. |

Form PCT/ISA/210 (second sheet) (January 2015)

**INTERNATIONAL SEARCH REPORT**

| International application No. |
|---|
| **PCT/JP2022/029561** |

| Box No. I | Nucleotide and/or amino acid sequence(s) (Continuation of item 1.c of the first sheet) |
|---|---|

1.  With regard to any nucleotide and/or amino acid sequence disclosed in the international application, the international search was carried out on the basis of a sequence listing:

    a.  ☑ forming part of the international application as filed:

        ☑ in the form of an Annex C/ST.25 text file.

        ☐ on paper or in the form of an image file.

    b.  ☐ furnished together with the international application under PCT Rule 13*ter*.1(a) for the purposes of international search only in the form of an Annex C/ST.25 text file.

    c.  ☐ furnished subsequent to the international filing date for the purposes of international search only:

        ☐ in the form of an Annex C/ST.25 text file (Rule 13*ter*.1(a)).

        ☐ on paper or in the form of an image file (Rule 13*ter*.1(b) and Administrative Instructions, Section 713).

2.  ☐ In addition, in the case that more than one version or copy of a sequence listing has been filed or furnished, the required statements that the information in the subsequent or additional copies is identical to that forming part of the application as filed or does not go beyond the application as filed, as appropriate, were furnished.

3.  Additional comments:

    "The form of Annex C/ST.25 text file" above shall read as "the form of ST.26.".

Form PCT/ISA/210 (continuation of first sheet) (January 2015)

INTERNATIONAL SEARCH REPORT
Information on patent family members

International application No.

**PCT/JP2022/029561**

| Patent document cited in search report | | | Publication date (day/month/year) | Patent family member(s) | Publication date (day/month/year) |
|---|---|---|---|---|---|
| JP | 2012-130672 | A | 12 July 2012 | (Family: none) | |
| JP | 2017-42477 | A | 02 March 2017 | (Family: none) | |

Form PCT/ISA/210 (patent family annex) (January 2015)

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- JP 2018094401 A
- EP 1014176 A
- US 6992172 B
- WO 200485473 A
- WO 2008103041 A
- JP 2010519293 A
- JP 2010519252 A
- JP 2010518833 A
- JP 2010519251 A
- WO 2010128672 A
- WO 2010147109 A
- JP 2021162131 A
- JP 22029561 B